(19) Europäisches Patentamt
European Patent Office
Office européen des brevets

(11) **EP 1 511 854 B1**

(12) **EUROPEAN PATENT SPECIFICATION**

(45) Date of publication and mention
of the grant of the patent:
**29.07.2009 Bulletin 2009/31**

(51) Int Cl.:
**C12Q 1/37** (2006.01)

(21) Application number: **03755144.7**

(22) Date of filing: **27.05.2003**

(86) International application number:
**PCT/EP2003/005569**

(87) International publication number:
**WO 2003/100376 (04.12.2003 Gazette 2003/49)**

(54) **HEMATOLOGICAL ASSAY AND KIT**

HÄMATOLOGISCHES BESTIMMUNGSVERFAHREN UND KIT

DOSAGE HEMATOLOGIQUE ET TROUSSE ASSOCIEE

(84) Designated Contracting States:
**AT BE BG CH CY CZ DE DK EE ES FI FR GB GR HU IE IT LI LU MC NL PT RO SE SI SK TR**

(30) Priority: **29.05.2002 EP 02011945**

(43) Date of publication of application:
**09.03.2005 Bulletin 2005/10**

(73) Proprietor: **DSM IP Assets B.V.**
**6411 TE Heerlen (NL)**

(72) Inventors:
• **CALATZIS, Andreas**
**81673 Munich (DE)**
• **WILMER, Marianne**
**CH-4144 Arlesheim (CH)**

(74) Representative: **Braun, André jr. et al**
**Braunpat Braun Eder AG**
**Reussstrasse 22**
**Postfach**
**4015 Basel (CH)**

(56) References cited:
EP-A- 0 229 234    EP-A- 0 420 332
WO-A-99/39212    DE-A- 19 904 674
US-A- 4 070 245    US-A- 4 289 498
US-A- 4 508 644    US-B1- 6 207 399

• RIJKERS D T S ET AL: "DESIGN AND SYNTHESIS OF THROMBIN SUBSTRATES WITH MODIFIED KINETIC PARAMETERS" THROMBOSIS RESEARCH, TARRYTOWN, NY, US, vol. 79, no. 5/6, 15 September 1995 (1995-09-15), pages 491-499, XP002000556 ISSN: 0049-3848

• LOTTENBERG R ET AL: "THE ACTION OF THROMBIN ON PEPTIDE P-NITROANILIDE SUBSTRATES: HYDROLYSIS OF TOS-GLY-PRO-ARG-PNA AND D-PHE-PIP-ARG-PNA BY HUMAN ALPHA AND GAMMA AND BOVINE ALPHA AND BETA-THROMBINS" THROMBOSIS RESEARCH, TARRYTOWN, NY, US, vol. 28, no. 1, 1 October 1982 (1982-10-01), pages 313-332, XP000196089 ISSN: 0049-3848

• BAUGHMAN D J ET AL: "THROMBIN ACTIVATION RATE CONSTANT 1 STAGE CHROMOGENIC ASSAY FOR THE EXTRINSIC SYSTEM" THROMBOSIS RESEARCH, vol. 26, no. 1, 1982, pages 1-12, XP009000059 ISSN: 0049-3848

• DE PEURIOT M D ET AL: "ELECTROCHEMICAL ACTIVITY DETERMINATION OF TRYPSIN-LIKE ENZYMES 4. COUPLED ELECTROCHEMICAL AND SPECTROPHOTOMETRIC ASSAY OF THROMBIN USING THE SAME D PHENYLALANYL PIPECOLYL ARGININE 4 METHOXY-BETA NAPHTHYLAMIDE DI HYDRO CHLORIDE S-2421 SUBSTRATES" THROMBOSIS RESEARCH, vol. 22, no. 3, 1981, pages 303-308, XP009000121 ISSN: 0049-3848

• HEMKER H C ET AL: "THROMBIN GENERATION IN PLASMA: ITS ASSESSMENT VIA THE ENDOGENOUS THROMBIN POTENTIAL" THROMBOSIS AND HAEMOSTASIS, STUTTGART, DE, vol. 74, no. 1, 1 July 1995 (1995-07-01), pages 134-138, XP000195941 ISSN: 0340-6245

- KIRCHHOF B ET AL: "THROMBIN GENERATION IN PROTHROMBIN COMPLEX PREPARATIONS ITS EFFECT ON A CHROMOGENIC SUBSTRATE FIBRINOGEN AND PLATELETS" THROMBOSIS RESEARCH, vol. 59, no. 3, 1990, pages 541-552, XP009000058 ISSN: 0049-3848
- HEMKER H C ET AL: "CONTINUOUS REGISTRATION OF THROMBIN GENERATION IN PLASMA, ITS USE FOR THE DETERMINATION OF THE THROMBIN POTENTIAL" THROMBOSIS AND HAEMOSTASIS, STUTTGART, DE, vol. 70, no. 4, 1993, pages 617-624, XP000567560 ISSN: 0340-6245
- CAPPIELLO MARIO ET AL: "Kinetics of human thrombin inhibition by two novel peptide inhibitors (Hirunorm IV and Hirunorm V)." BIOCHEMICAL PHARMACOLOGY, vol. 52, no. 8, 1996, pages 1141-1146, XP001096097 ISSN: 0006-2952

**Description**

Background

The present invention relates to a method of assessing the coagulation potential, or thrombin-forming potential of a sample of blood or plasma.

Rijkers et al. ("Design and Synthesis of Thrombin Substrates with Modified Kinetic Parameters", Thrombosis Research, 1995, Vol. 79, No. 5/6, pages 491-499) describes a research study of a number of chromogenic thrombin substrates.

Lottenberg et al. ("The Action of Thrombin On Peptide P-Nitroanilide Substrates: Hydrolysis of Tos-Gly-Pro-Arg-Pna and D-Phe-Pip-Arg-Pna by Human Alpha and Gamma and Bovine Alpha and Beta-Thrombins', Thrombosis Research, 1982, Vol. 28, No. 1, Pages 313-332) describes a study of the activity of various thrombin solutions on various chromogenic thrombin substrates.

Baughman et al. ("Thrombin Activation Rate Constant 1 Stage Chromogenic Assay For the Extrinsic System" Thrombosis Research, 1982, Vol. 26, No. 1, pages 1-12) describes a chromogenic clotting assay using tissue thromboplastin as an activator and a thrombin substrate as an indicator.

De Peuriot et al. ("Electrochemical Activity Determination of Trypsin-Like Enzymes 4. Coupled Electrochemical and Spectrophotometric Assay of Thrombin Using the Same D Phenylalanyl Pipecolyl Arginine 4 Methoxy-Beta Naphthylamide Di Hydro Chloride S-2421 Substrates", Thrombosis Research, 1981, Vol. 22, No. 3, pages 303-308) describes a study using amperometric detection of an electroactive species released from an electrogenic substrate through thrombin digestion.

Gradipore (WO 99 39212) describes a coagulation assay comparing rates of coagulation against standards, using snake venoms.

Hemker et al. (EP-A-O 420 332) describes the endogenous thrombin potential (ETP) method wherein the thrombin substrate is not consumed and wherein the addition of fibrinogen polymerisation inhibitor is optional.

HaemoSys (DE-A-19904674) describes a method for quantification of hirudin and other direct thrombin inhibitors. Activation of prothrombin is carried out using thrombin itself as activator and a chromogenic thrombin substrate is used to measure the inhibition.

Boehringer Mannheim GmbH (EP-A-0229234) describes a method which first produces a plasma fraction rich in Protein C and then mixes this plasma fraction with a normal plasma pool and performs an activated partial thromboplastin time (aPTT) assay in order to detect the inhibition of the clotting by the protein C from the sample.

It is essential for survival that a wound stops bleeding and that childbirth be compatible with survival of the mother, i.e. that the body possesses an adequate mechanism for hemostasis. If however arrest of blood flow occurs in intact vessels, normal circulation is impaired, which is deleterious to normal function. Tissues and organs can die when blood supply is arrested for too long. Nature has provided an admiringly efficient and extremely complicated mechanism, the hemostatic system, to ensure the seemingly contradictory functions: adequate blood flow in normal vessels and prompt arrest of bleeding in damaged ones. It is not surprising that the hemostatic mechanism is a very complicated and extremely fine tuned one, replete with checks and balances (Hemker H C, Beguin S. Phenotyping the clotting system. Thromb Haemost 2000; 84: 747-51).

Hemostasis is brought about by the interplay between the minute blood cells, the blood platelets, and a set of proteins of the blood plasma, the coagulation system. Having past a damaged part of the vessel wall, platelets stick to the bare tissue in the wound and create a scenery in which blood can clot without the clot being washed away by the flowing blood. The interactions between the wound and the blood lead to the formation of thrombin. Thrombin is responsible for a whole concert of reactions, of which the actual clotting, i.e. solidification of the blood is only one: Thrombin activates the platelets and acts on the cell of the vessel wall. Activated platelets, in their turn, foster thrombin formation. Thrombin partakes in a whole set of positive and negative feedback reactions. (Hemker et al US 5,192,689)

Essential to the understanding is the concept of proenzyme-into-enzyme conversion. An enzyme is a protein capable to enhance a very specific chemical reaction. Proteolytic enzymes are proteins that can cut other proteins in pieces. They can therefore be extremely dangerous and usually are formed and transported in the body as proenzymes. Proenzymes are usually larger than enzymes and cannot, as such, do any harm.

Only when they are cut at a specific place does their enzyme character appear. E.g. enzymes that are to digest the proteins of our food are formed as proenzymes in the pancreas and are only activated when secreted in the gut. The clotting mechanism is based on an ordered series of proenzyme - enzyme conversions. The first one of the series is transformed into an active enzyme that activates the second one, which activates the third one. In this way a few molecules in the beginning of the series create an explosion of the final active enzyme: thrombin. This mechanism is often referred to as the coagulation cascade.

Thrombin, in the blood, does not live long; otherwise the slightest wound could make all the blood clot. Its survival time in plasma is only a few minutes, due to the fact that it is bound by antithrombins, suicidal plasma proteins that inactivate thrombin by binding to it irreversibly. Antithrombins also act as regulatory proteins which can inactivate the first traces of thrombin before they can augment thrombin generation by the positive feedback reactions.

The cascade sequence only explains the explosive formation of thrombin, however, clotting is not as simple as that, the output is finely tuned by a web of positive and negative feedback reactions. Coagulation starts when a vessel is wounded. A vessel is wrapped in a layer that is full of cells containing Tissue Factor (TF) (Mann KG: Biochemistry and physiology of blood coagulation. Thromb Haemost 1999 Aug;82(2):165-74). After injury this TF is exposed to blood, it binds with factor VIIa to form the first enzyme: TF:VIIa. This activates factor X to Xa, which in turn forms thrombin. All the plasma proteins involved in coagulation are clotting factors, indicated by Roman numerals. When a proenzyme is activated into an enzyme, an "a" is added to the number. Factor Xa is not a very efficient enzyme; it needs the help of activated factor V (a helper protein or cofactor) that helps to speed up prothrombin activation a thousand fold. Both factors bind to a phospholipid surface that also binds prothrombin and thus favours the meeting of substrate and enzyme. (Xi M, Beguin S, Hemker H C. The relative importance of the factors II, VII, IX and X for the prothrombinase activity in plasma of orally anticoagulated patients. (Thromb Haemost 1989; 62: 788-91)

This offers the possibility to regulate the function of factor Xa, and hence thrombin production in space and time. Factor V is activated by thrombin into factor Va. Xa and Va bind onto a phospholipid surface and form the prothrombinase complex which very efficiently converts more prothrombin into thrombin. Thrombin also binds to thrombomodulin. This complex converts protein C (again a proenzyme) into its activated (enzyme) form: APC. APC in turn attacks factor Va and inactivates it (by converting it to factor Vi). (Rosing J, Hemker HC, Tans G. Molecular biology and pathophysiology of APC resistance: current insights and clinical implications. Semin Thromb Hemost. 1998;24(4):329-35.)

VIIa:TF not only activates factor X but also factor IX (nine). Thrombin also activates factor VIII that, just like factor V, is a helper protein. Factor VIIIa and IXa form a complex called tenase, that produces more X. Like factor Va, factor VIIIa is degraded by activated protein C. Like the Xa-Va complex, the IXa-VIIIa complex only functions well when adsorbed onto a phospholipid surface.

Blood contains platelets (thrombocytes). As soon as they find an injury they bind to the exposed tissue (collagen) and, by this binding and the contact with thrombin, they activate. This results in a change of the surface of the platelets, that now becomes able to bind clotting factors such as the couples Xa-Va and IXa-VIIIa. This binding is essential for their proper functioning. The platelet surface serves as a two-dimensional space where the clotting factors can find each other and more easily perform their reactions. This adsorption again increases thrombin formation by about a thousand fold. So without such a surface clotting will not occur. This provides the Localisation Dimension: clotting only occurs where it is needed and remains contained to that area (Beguin S, Keularts I. On the coagulation of platelet-rich plasma. Physiological mechanism and pharmacological consequences. Haemostasis 1999; 29: 50-7).

The result of this all is that a complicated web of positive and negative feedback loops governs thrombin production. These mechanisms are so complicated as to make it (even theoretically) impossible to describe them in a mathematical model. Therefore it becomes extremely difficult to predict with any accuracy how the system will quantitatively answer to changes to one or more of its reactants.

Drugs that are designed to inhibit thrombosis by selectively attacking one single enzyme may in practice work out completely different than expected. The consequence is that the coagulation potential of the blood has to be measured rather than to try to predict it.

The conventional way of assessing the hemostatic system is to activate hemostasis (using different activators) and to assess the time it takes for blood or plasma until clotting is detected ("clotting time"). Alternatively the concentration of certain components is determined or the activity of certain components is assessed in artificial isolated systems (single factor activities). By all these approaches the dynamics of thrombin activation are not directly assessed. Clotting times only represent the lag phase before thrombin generation starts and therefore only tell a minor part of the story. The extent of the hemostatic- thrombotic reaction is also critically determined by the dynamics of thrombin formation.

In the past different strategies for quantifying the dynamics of thrombin formation have been developed. Most known are the techniques developed by Hemker and Beguin (US Patent No. 5,192,689) et al and modifications of these methods by other authors.

The method of Hemker

The method of Hemker relies on the determination of the so-called "endogenous thrombin potential" (ETP) in a sample of either clotting blood or plasma.

A thrombin formation activator is added to the sample together with a thrombin substrate, wherein the amount and also the kinetic properties of said thrombin substrate are chosen such that the amount of thrombin generated in the sample cannot completely consume said thrombin substrate. Thrombin formed during the clotting reaction consumes said substrate, producing a conversion product. The amount of conversion product is determined, and from this the endogenous thrombin potential in the sample is calculated (Hemker H C, Wielders S, Kessels H, Beguin S. Continuous registration of thrombin generation in plasma, its use for the determination of the thrombin potential. Thromb Haemost 1993; 70: 617-24).

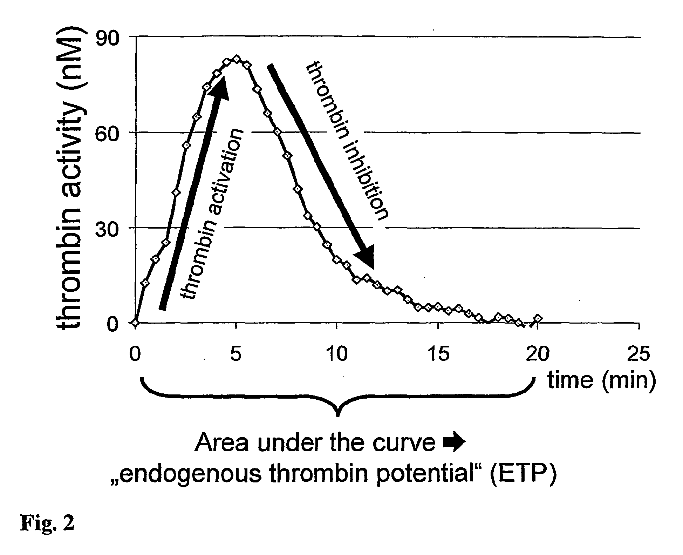

Typically the ETP is calculated based on the area under the curve of the thrombin activity (Fig. 2). The thrombin activity is calculated based on the first derivative of the production of the conversion product of the thrombin substrate.

The ETP method by Hemker relies on the continuous assessment of thrombin formed *in vitro*, - from its formation until it is inhibited by the endogenous inhibitory substances in the blood or by added inhibitors in the reagent (Fig. 2). The ETP method is based on the concept that the hemostatic activity that develops in a wound or thrombus is essentially dependent upon the number of "man-hours" of thrombin that can develop in blood. According to this concept the amount of thrombin that generates as well as the length of time that it is active both count. The amount of work that can potentially be done by thrombin is reflected in the area under the curve that describes the concentration of thrombin in time during clotting, that is, the ETP. Another important property is the time it takes until thrombin formations starts, i.e. the lag time. This also happens to be the clotting time, because the clot appears when roughly 1% of thrombin is formed. A lot of information is missed if only the clotting time is observed as a determinant of coagulability: after the clot formation most of the thrombin action is still to come (Hemker H C, Beguin S. Thrombin generation in plasma: its assessment via the endogenous thrombin potential. Thromb Haemost 1995; 74: 134-8).

The following strategies are commonly used in hematology, e.g. for assessing the ETP:

Use of chromogenic thrombin substrates

Chromogenic substrates are peptides which are coupled to chromophoric groups. They are usually applied in aqueous solution. When these chromophoric groups are split from the peptide by enzymatic action the optical density of the solution rises and this can be detected photometrically. Chromogenic substrates are used in many diagnostic methods for the assessment of the activity of certain enzymes. The commonly used chromogenic substrates for thrombin are readily split by thrombin action and are thus rapidly consumed by its action. For the use in the ETP method chromogenic substrates have been applied which are slowly converted by thrombin. These are either substrates which were originally developed for other enzymes (e.g. the substrate S-2222, which was developed for the assessment of FXa activity) or substrates developed especially for the assessment of the ETP (Rijkers D T, Hemker H C, Tesser G I. Synthesis of peptide p-nitroanilides mimicking fibrinogen- and hirudin-binding to thrombin. Design of slow reacting thrombin substrates. Int J Pept Protein Res 1996; 48: 182-93.).

The chromophors (e.g. p-nitro-aniline [p-NA]) used in the chromogenic substrates are typically assessed using light with a wavelength of 405 nm. Both fibrin and platelets in aqueous solutions interfere with the assessment of the release of the chromophors of chromogenic substrates at 405 nm. When thrombin is formed it splits two pieces of the fibrinogen molecule (so called fibrino-peptides A and B). The resulting molecule is called fibrin. It spontaneously polymerises to form fibrin polymers (fibrin strands), which are the covalently cross-linked by factor XIIIa (a clotting factor which is also activated by thrombin). This mechanism (fibrin formation and polymerisation) is an integral part of the hemostatic process in the body.

In order to assess the ETP using chromogenic substrates the sample must either be depleted from fibrinogen (defibrination) before the analysis or substances which interfere with fibrin polymerisation must be added (Wielders S, Mukherjee M, Michiels J, Rijkers D T, Cambus J P, Knebel R W, Kakkar V, Hemker H C, Beguin S). The routine determination of the endogenous thrombin potential, first results in different forms of hyper- and hypocoagulability. Thromb Haemost 1997; 77: 629-36). The defibrination of the sample can be performed using snake venoms which split fibrinogen. The resulting fibrin clot can be removed from the plasma by centrifugation or can be removed manually. However this requires a separate step for the preparation of the sample, which can not be performed by the standard laboratory analysers. As a separate step which has to be performed by the laboratory staff instead of the automated equipment it significantly enhances the cost of such an analysis. The defibrinated sample cannot be used for the standard analysis of the coagulation system, but can only be used for the ETP method. In addition it is also unclear whether the removal of the artificially produced fibrin clot also extracts any relevant substances from the plasma sample that might be relevant for the function of the coagulation cascade (Kumar R, Beguin S, Hemker H C). The influence of fibrinogen and fibrin on thrombin generation is an evidence for feedback activation of the clotting system by clot bound thrombin. (Thromb Haemost 1994; 72: 713-21).

Substances are known that inhibit fibrin polymerisation. The most commonly used substance is the peptide H-Gly-Pro-Arg-Pro-OH AcOH. The use of this peptide in the ETP method has been introduced by Stuerzebecher et al and has the major advantage that the sample has not to be defibrinated before the analysis (Prasa D, Svendsen L, Sturzebecher J. Inhibition of thrombin generation in plasma by inhibitors of factor Xa. Thromb Haemost 1997 Oct;78(4):1215-20; Prasa D, Svendsen L, Sturzebecher J). The ability of thrombin inhibitors to reduce the thrombin activity generated in plasma on extrinsic and intrinsic activation. Thromb Haemost 1997 Mar;77(3):498-503).

However quite high concentrations of H-Gly-Pro-Arg-Pro-OH AcOH must be applied and the inhibition of fibrin polymerisation is often incomplete, especially in samples with high fibrinogen concentrations. As fibrinogen is a so-called acute phase protein it can reach very high concentrations in the plasma of patients (up to 10 g/l). Thus many patients whose ETP might be relevant have elevated fibrinogen concentrations which can disturb the assessment of the ETP using fibrin polymerisation inhibitors.

Use of fluorogenic substrates

Another strategy for assessing the ETP - which is today more popular than the chromogenic method - is the use of fluorogenic substrates (Hemker H C, Giesen P L, Ramjee M, Wagenvoord R, Beguin S). The thrombogram: monitoring thrombin generation in platelet-rich plasma. (Thromb Haemost 2000; 83:589-91). Fluorogenic substrates are analogous to chromogenic substrates. The difference is that the substrates release upon enzymatic action a group which can be determined using a fluorometer. Fluorometers have a high sensitivity for detecting low amounts of substances with fluorescent properties. Fluorometric assessment of the ETP also has the advantage that fibrin or platelets do not interfere with the analysis. I.e. no measures have to be performed for defibrinating the sample or for inhibiting fibrin polymerisation. In addition platelet-rich plasma can be used for the analysis, which allows the assessment of the interaction of the plasmatic factors (enzymes) and platelets during thrombin formation (Keularts I M, Hamulyak K, Hemker H C, Beguin S. The effect of DDAVP infusion on thrombin generation in platelet-rich plasma of von Willebrand type 1 and in mild haemophilia A patients. Thromb Haemost 2000; 84: 638-42).

However the use of fluorogenic substrates has also significant disadvantages: The standard laboratory equipment used for analysis of the coagulation system does not support fluorometric analysis. Thus the analysis requires additional expensive instrumentation (fluorometer). This produces significant costs and is a major obstacle for the introduction of this method. In addition the trend of the last decade has been to implement all coagulation tests wherever possible on one analyser in order to simplify the testing procedure and minimise labour costs. The use of a separate instrument for the ETP method reduces significantly its applicability as a routine method.

The fluorometers currently used for the ETP method do not provide an automated pipetting of the ETP procedure, thus for performing the procedure several manual pipetting steps are required, which again enhances the cost of the procedure and complicates the introduction of the method in routine laboratories.

General aspects of the ETP method by Hemker

As mentioned the ETP method by Hemker and Beguin (US-Patent No. 5,192,689) requires the use of a slow substrate for thrombin, i.e. a substrate that has a high $K_M$ and is thus slowly converted by thrombin formed during by the coagulation cascade. Hemker explains that it is necessary to use a thrombin substrate with a high $K_M$ in order to minimise artificial effects of the substrate on the coagulation cascade. Most coagulation factors are- like thrombin -serine proteases and have homologies to thrombin. According to Hemker in order to assess thrombin formation in a valid way one has to use a substrate with a high $K_M$ which will thus not lead to a major interference with the coagulation cascade.

However the use of a slow thrombin substrate leads to a relatively weak signal. This complicates the precise assessment of the "lag phase" i.e. the time from the start of the analysis until the detection of free thrombin in the sample-reagent mixture.

The use of a slow substrate together with the requirement to assess thrombin activation and inactivation lead to very long measuring times (typically 30 minutes) and to the need of high substrate concentrations, which both enhance the costs of the analysis. For an analysis in a routine setting measuring times of 30 minutes have two negative effects: The turn-around time is significantly enhanced, thus limiting the usefulness of the assay for immediate therapeutic decisions. The analyser is blocked for a significant time period.

The ETP method by Hemker is based on the determination of the area under the curve (AUC) of thrombin activity, *i.e.* on the "man hours" that are performed by the thrombin molecules from their activation to their inactivation. Several inhibitory substances inactivate thrombin *in vitro.* One is antithrombin, an inhibitor which forms irreversible complexes with thrombin and other serine proteases and whose action is enhanced by heparins. Another is heparin co-factor II, a more specific inhibitor, whose anti-thrombin action is also enhanced by heparins. Thrombin is also inhibited by less specific inhibitors in plasma. One of the most important is alpha-2-macroglobulin (alpha2-m). Alpha-2-m does not bind to the active centre of thrombin (as most other inhibitors do), but incorporates thrombin like a cage. Thrombin which is bound to alpha2-m loses its biological activities: it does not activate platelets and does not split fibrinogen, factor V, factor VIII, factor XI or factor XIII. However it still can split the (small) chromogenic or fluorogenic substrates and thus mimic the activity of active thrombin. This disturbs the correct assessment of the ETP according to Hemker (Rijkers DT, Wielders SJ, Beguin S, Hemker HC: Prevention of the influence of fibrin and alpha2-macroglobulin in the continuous measurement of the thrombin potential: implications for an endpoint determination of the optical density; Thromb. Res. 1998 Feb 15;89(4):161-9). As the concentration of alpha2-m in the sample varies from individual to individual and is also affected by disease states, complex algorithms are required in order to subtract the activity of alpha2-m-bound thrombin from the total thrombin activity determined (Kessels H, Willems G, Hemker HC. Analysis of thrombin generation in plasma. Comput Biol Med 1994 Jul; 24(4):277-88 ).

Methods for the assessment of the thrombin formation in fluids using separate aliquots ("sub-sampling")

In many scientific studies methods are applied that assess the thrombin generation of hemostatic processes using a process called 'sub-sampling'. Examples are given in: Beguin S, Lindhout T, Hemker HC. The effect of trace amounts of Tissue Factor on thrombin generation in platelet rich plasma, its inhibition by heparin. Thromb Haemost 1989 Feb 28;61(1):25-9, Kessels H, Beguin S, Andree H, Hemker HC. Measurement of thrombin generation in whole blood--the effect of heparin and aspirin. Thromb Haemost 1994 Jul; 72(1):78-83, Reverter JC, Beguin S, Kessels H, Kumar R, Hemker HC, Coller BS. Inhibition of platelet-mediated, Tissue Factor-induced thrombin generation by the mouse/human chimeric 7E3 antibody. Potential implications for the effect of c7E3 Fab treatment on acute thrombosis and "clinical restenosis". J Clin Invest 1996 Aug 1;98(3):863-74, Butenas S, van't Veer C, Mann KG. "Normal" thrombin generation. Blood 1999 Oct 1;94(7):2169-78, van 't Veer C, Golden NJ, Kalafatis M, Simioni P, Bertina RM, Mann KG. An in vitro analysis of the combination of haemophilia A and factor V (LEIDEN). Blood 1997 Oct 15;90(8):3067-72

In these and many other studies a blood or plasma sample is reacted with an activator of hemostasis.

During the activation of coagulation small aliquots are repeatedly extracted from the sample and placed in a buffer solution which contains a chromogenic thrombin substrate (this process is called "sub-sampling"). In each aliquot the thrombin activity is assessed separately. This procedure produces significant effort, as up to 80 samples are analysed for each experiment and is therefore not applicable for routine use.

Thrombin substrates are also used in various other laboratory methods, e.g. synthetic substrates for thrombin are used in different applications for the analysis of hemostasis (Fareed J, Messmore HL, Walenga JM, Bermes EW Jr. Synthetic peptide substrates in hemostatic testing. Crit Rev Clin Lab Sci. 1983;19(2):71-134). Among these applications are methods that assess the antithrombin activity (Blomback M, Blomback B, Olsson P, Svendsen L: The assay of antithrombin using a synthetic chromogenic substrate for thrombin. Thromb Res 1974 Nov;5(5):621-32), the heparin concentration (Choo IH, Didisheim P, Doerge ML, Johnson ML, Bach ML, Melchert LM, Johnson WJ, Taylor WF. Evaluation of a heparin assay method using a fluorogenic synthetic peptide substrate for thrombin. Thromb Res Suppl. 1982 Jan 1-15;25(1-2):115-23.), the thrombin or prothrombin activity (Hitomi Y, Kanda T, Niinobe M, Fujii S. A sensitive colorimetric assay for thrombin, prothrombin and antithrombin III in human plasma using a new synthetic substrate. Clin Chim Acta 1982 Mar 12;119(3):157-64), and methods that assess the anticoagulant drug hirudin and other direct thrombin inhibitors (Griessbach U, Sturzebecher J, Markwardt F: Assay of hirudin in plasma using a chromogenic thrombin substrate. Thromb Res Suppl. 1985 Jan 15;37(2):347-50.). Using these methods the activity of the thrombin or prothrombin in the sample is specifically assessed or the inhibition of added thrombin by endogenous or exogenous anticoagulants is determined.

Further methods have been described that apply thrombin substrates in order to assess the Prothrombin Time: (US4784944 (Kolde): Prothrombin Time determining reagent and methods of using and preparing the same) (Ohki M, Tanaka S, Uchida K, Yamaguchi T, Kato H. A basic study on a highly sensitive automated method for hypercoagulable state in plasma, fluorogenic Prothrombin Time method. Rinsho Byori 1993 Oct;41(10):1153-8) (US4289498 (Baughman): One-stage prothrombin assay and compositions useful therein).

In US4784944 and a method described by Ohki et al (Ohki M, Tanaka S, Uchida K, Yamaguchi T, Kato H. Rinsho Byori 1993 Oct; 41(10): 1153-8) a thrombin substrate is applied for the assessment of the onset of thrombin formation following activation of coagulation by Tissue Factor. These methods were not developed for assessing the amount and velocity of the thrombin formed during the reaction (as in the inventive assay), but only in order to assess the time point of free thrombin formation.

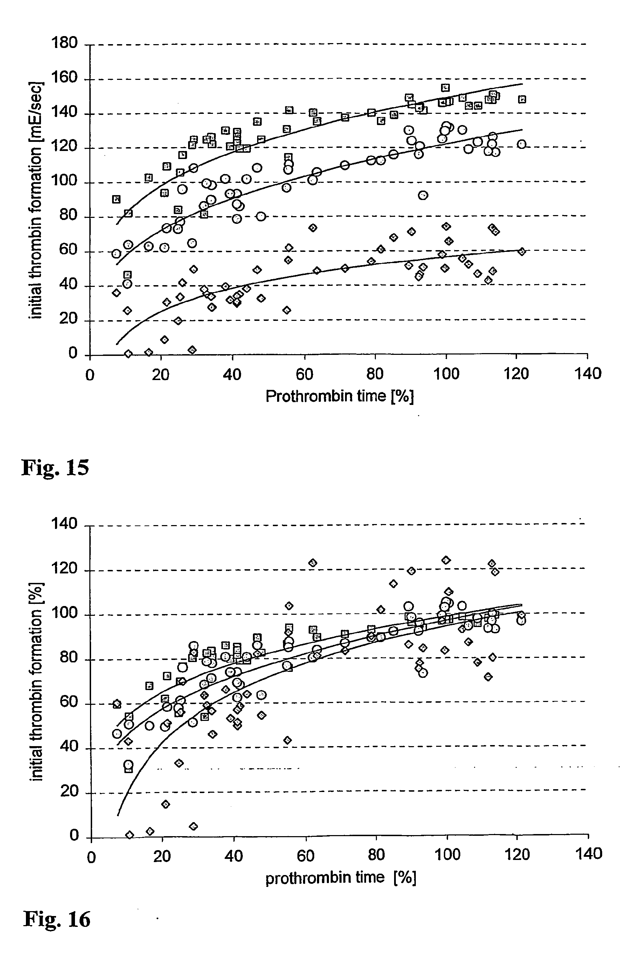

In US4289498 a method for the determination of the Prothrombin Time is described, which is based on the determination of the exponential rate of the thrombin production in thromboplastin-treated recalcified plasma. For the determination of the rate of thrombin production a thrombin substrate is applied. In this source the thrombin substrate is not used in order to quantify thrombin formation itself, but as a indirect measure for the Prothrombin Time in a highly diluted assay. Using the diluted assay and the addition of a so-called "serum reagent", which includes various activated coagulation factors, the thrombin formation in the patient sample is modified when compared to the physiological situation. In contrast the inventive assay assesses the thrombin formation using a much less artificial setting. The Baughman source does not teach the use of a relatively small amount of a fast chromogenic substrate for assessing the initial thrombin formation by the hemostatic mechanisms in the sample. In contrast on page 12 line 59-69 Baughman states that the thrombin substrate is preferably applied in stochiometric excess compared to the amount of plasma and that the upper limit of the concentration of the chromogenic substrate is simply the solubility of the substrate in the mixture, which shows that the advantages of the inventive method had not been recognised. In the preferred embodiment in the Baughmann source the chromogenic substrate is used in a concentration of 11.85 mM in relation to the added plasma volume. In the inventive method the chromogenic substrate is preferably used in a concentration of only 250 $\mu$M in relation to the same volume of added plasma. As shown later in Fig. 15 and Fig. 16 the inventive assay does not simply assess the Prothrombin Time, as there is considerable variation between the inventive assay and this routine method.

Summary of the prior art

As aforementioned the conventional way of assessing the hemostatic system is for instance to activate hemostasis and to assess the "clotting time". Alternatively the concentration of certain components is determined or the activity of certain components is assessed in artificial isolated systems (single factor activities). By all these approaches the dynamics of thrombin activation are not directly assessed. "Clotting times" only represent the lag phase before thrombin generation starts.

Furthermore, the use of chromogenic substrates for assessing the "endogenous thrombin potential" (ETP) of a defibrinated sample requires a separate step for the preparation of the sample, which can not be performed by the standard laboratory analysers. Additionally, the defibrinated sample cannot be used for the standard analysis of the coagulation system, but can only be used for the ETP method. In addition it is also unclear whether the removal of the artificially produced fibrin clot also extracts any relevant substances from the plasma sample that might be relevant for the function of the coagulation cascade. The peptide H-Gly-Pro-Arg-Pro-OH AcOH which inhibits fibrin polymerisation can be used in the ETP method. However, quite high concentrations of H-Gly-Pro-Arg-Pro-OH AcOH must be applied and the inhibition of fibrin polymerisation is often incomplete, especially in samples with high fibrinogen concentrations.

The use of fluorogenic substrates has also significant disadvantages: The standard laboratory equipment used for analysis of the coagulation system does to support fluorometric analysis. Thus the analysis requires additional expensive instrumentation. Furthermore, the performance of an assay with fluorogenic substrates requires several manual pipetting steps which again enhances the cost of the procedure and complicates the introduction of the method in routine laboratories.

The use of a slow thrombin substrate according to the method of Hemker leads to a relatively weak signal. This complicates the precise assessment of the "lag phase".

The use of a slow substrate together with the requirement to assess thrombin activation and inactivation lead to very long measuring times and to the need of high substrate concentrations, which both enhance the costs of the analysis.

Summarising, all currently available methods for quantifying the thrombin formation during coagulation processes have several disadvantages, which have limited their use up until now to non-routine research applications.

Thus the great challenge for the present invention is to overcome problems and disadvantages associated with the routine use of prior art methods and to provide a fast and precise direct method for assessing the coagulation potential of a sample of blood or plasma. This is achieved by observing the initial dynamics of thrombin formation in a sample.

Definitions

As used herein the following terms will have the meanings indicated:

Antithrombins are suicidal plasma proteins which inactivate thrombin by binding to is irreversibly. They act as regulatory proteins which can inactivate the first traces of thrombin before they can augment thrombin generation by the positive feedback reactions. The action of some Antithrombins is enhanced by heparins.

Clotting time is the time it takes for blood or plasma until clotting is detected after induction of hemostasis.

Chromogenic substrates are peptides which are coupled to chromophoric groups. When these chromophoric groups are split from the peptide by enzymatic action the optical density of the solution rises and this can be detected photometrically.

Fluorogenic substrates can be used analogous to chromogenic substrates. The difference is that the substrates release upon enzymatic action a group which can be determined using a fluorometer.

Activator of the coagulation system Several substances are known that can activate hemostasis. Activators of the plasmatic coagulation system induce the cleavage of coagulation factors to their active forms. E.g. Tissue factor forms a complex with activated factor VII. This complex activates factor X and factor IX. Other substances (e.g. collagen) activate the blood platelets. Upon activation platelets secrete substances, which activate coagulation and present adhesive receptors on their surface. In addition the composition of the platelet surface changes in order to allow the attachment of clotting factors.

Thrombin substrates are substances, which are split by thrombin and release groups which can be quantitatively detected. Examples are chromogenic thrombin substrates (the conversion product is detected photometrically) or fluorogenic thrombin substrates (the conversion product is detected using fluorometric analysis).

A coagulation accelerant is a material or substance or mixture of such which greatly speeds up the rate of thrombin formation. Phospholipids and calcium ions are coagulation accelerants as they promote the formation of complexes of coagulation factors. In these complexes (e.g. the prothrombinase complex) the activation of coagulation factors is significantly accelerated.

The first derivative of a curve reflects the gradient of the curve during the reaction, the reaction velocity. The first derivative of the optical density curve of the sample-reagent mixture during the analysis of coagulation using a thrombin substrate reflects the thrombin activity during time.

$K_M$ Abbreviation for the Michaelis constant which may be defined as the substrate concentration at which the reaction velocity is half of the maximal velocity. It is an intrinsic property of the enzyme related to the binding constant for forming the particular enzyme-substrate complex. In the present context the enzyme is thrombin.

Summary of the invention

According to one aspect, the invention provides a method of assessing the coagulation potential of a sample of blood or plasma comprising:

a) mixing a blood or plasma sample with at least one activator of the blood coagulation system and a thrombin substrate, and if necessary incubating the mixture,
b) incrementally or continuously determining the release of the conversion product of the thrombin substrate, and
c) calculating at least one value indicative of the coagulation potential,

wherein:

i.) the quantity of substrate is sufficiently small to be completely consumed by a specified quantity of normal blood or plasma,
ii.) the amount and kinetic properties of the substrate are selected such that said consumption takes place within a range from 5 to 600 seconds,
iii.) the said value calculated is dependent on the velocity of consumption of the substrate.

Preferably the value determined is the maximum rate of consumption of the substrate, which is dependent upon thrombin-forming potential of the sample.

Thus, in general terms, a fast acting substrate is selected and used in a low concentration relative to the sample, in contrast to the method of Hemker described above. The substrate may conveniently be selected by its Michaelis constant ($K_M$) for the substrate-thrombin reaction which is a property generally known, and the inventive selection [ii) above] could if necessary be alternatively be expressed in terms of this constant. Preferably the thrombin substrate has a $K_M$ of 200 $\mu$M or less, more preferably 100 $\mu$M or less, and most preferably 50 $\mu$M or less.

The thrombin substrate may be a chromogenic, a fluorogenic, or an amperogenic thrombin substrate or other substrate for detecting the thrombin activity. In a preferred embodiment the chromogenic substrate is selected from one or more of:

H-D-CHG-Ala-Arg-pNa·2AcOH,
H-D-Phe-Pip-Arg-pNA·2HCl,
Boc-Asp(OBzl)-Pro-Arg-NH-Mec,
pyroGlu-Pro-Arg-pNA·HCl and Bz-Phe-Val-Arg-pNA.

The method may further comprise or utilise a substance which interferes with the fibrin gelation, preferably a fibrin polymerisation inhibitor, such as H-Gly-Pro-Arg-Pro-OH AcOH e.g. in a concentration of 0.1-20 mg/ml, preferably 5 mg/ml.

Furthermore, the method may comprise or utilise a coagulation inhibitor. Examples for suitable coagulation inhibitors are Protein C activators (e.g. Protac), Tissue Factor Pathway Inhibitor (or equivalent substances), inactivated factor VIIa (or equivalent substances), Activated Protein C (or equivalent substances), platelet inhibitors (e.g. prostacyclin), FVIII inhibitor (or equivalent substances), FIX inhibitor (or equivalent substances), heparins and/or heparinoids, direct thrombin inhibitors, direct Factor Xa inhibitors and serine protease inhibitors (e.g. aprotinin).

The method may also comprise or utilise coagulation accelerant. Suitable coagulation accelerants are phospholipids of natural or synthetic origin or analogous substances and calcium chloride or other sources of divalent cations. Activators of the coagulation system which may be used according to the present invention are Tissue Factor or analogous substances, activators of factor X (e.g. snake venoms), activators of factor V (e.g. snake venoms), prothrombin activators (e.g. snake venoms), contact phase activators (e.g. kaolin), activated coagulation factors or analogous substances and platelet activators (e.g. collagen). A preferred activator of the coagulation system is recombinant Tissue Factor which may be used in a concentration of 2-200 ng/ml. Another preferred activator of the coagulation system, aluminium silicate, may be used in a concentration of 0.5-10 g/l.

Furthermore, the conversion product of the substrate can conveniently be detected optically at 405 nm suiting most instruments in use.

Other preferred features of the invention will be found set out in the claims.

Brief description of the drawings

Figure. 1. is a schematic diagram showing the complex interactions involved in the inventive assay,

Figure 2. (Prior art) is a diagram showing the assay of Hemker to obtain the endogenous thrombin potential (ETP),

Figure 3. (Prior art) is a graph showing the rise of the optical density by the conversion of a slow chromogenic thrombin substrate according to the prior art at different substrate concentrations,

Figure 4. (Prior art) is a graph showing the first derivative of the reaction curves of Figure 3,

Figure 5. is a graph showing the rise of the optical density and strong signal obtained by the conversion of a fast chromogenic thrombin substrate according to the inventive assay at different substrate concentrations,

Figure 6. is a graph showing the first derivative of the reaction curves of Figure 5,

Figure 7. is a graph showing the effect of the activation on the inventive assay of different concentrations of recombinant Tissue Factor (concentration shown in the figure in ng/ml),

Figure 8. is a graph showing the first derivative of the reaction curves shown in Figure. 7,

Figure 9. (Prior art) is a graph illustrating the effect of optical anomalies due to fibrin gelation phenomena,

Figure 10. (Prior art) is a graph showing the first derivative of the reaction curve shown in Figure 9,

Figure 11. is a graph showing the correlation of the onset of clotting (detected by fibrin formation) and the onset of thrombin formation detected by the inventive assay,

Figure 12. is a graph showing the effect of decreasing activities of the coagulation factor FVIII (activities shown in the figure) on the inventive assay,

Figure 13. is a graph showing the correlation of the factor VIII activity and the initial thrombin formation (assessed according to the inventive assay) in hemophilia A patients and healthy volunteers,

Figure 14. is a graph showing the effect of the addition of FVIII to hemophilia A patients on the initial thrombin formation (assessed using the inventive method),

Figure 15. is a graph showing the correlation of the initial thrombin formation (assessed according to the inventive method) and by the Prothrombin Time method,

Figure 16. is a graph showing the correlation of the initial thrombin formation (assessed according to the inventive method), expressed to the Prothrombin Time.

Detailed description of the invention

Quite unexpectedly we found that an assay system which uses in combination an activator of the plasmatic coagulation system, a blood or plasma sample and a fast thrombin substrate in a relatively small concentration (kinetics and amount of the thrombin substrate are chosen such as it will be typically consumed within 5-600 seconds after thrombin activation) allows a much more precise, inexpensive and fast route to the assessment of the coagulation or thrombin-forming potential of a test sample of blood or plasma than previously applied methods.

The assay system is less dependent on the thrombin inhibition phase, than previous methods for the assessment of thrombin activation, which has several advantages and enhances the physiological relevance of the assay.

When a chromogenic thrombin substrate is applied a fibrin polymerisation inhibitor (or an analogous substance) is normally applied in order to suppress fibrin gelation which would interfere with the conversion of the chromogenic substrate. However, if fluorogenic detection is applied (or other detection methods, which are not disturbed by the fibrin gelation) then this component is not required for the inventive assay.

For performing the assay according to the present invention the following reagents are preferably used:

an activator of the plasmatic coagulation system (e.g. recombinant Tissue Factor in a concentration of 200 ng/ml sample)

a chromogenic substrate with a $K_M$ of 200 $\mu$M or less (e.g.

H-D-CHG-Ala-Arg-pNa·2AcOH at a concentration of 250 $\mu$M/ml sample)

optionally a coagulation accelerant (e.g. phospholipids at a concentration of 50 $\mu$g/ml sample, and CaCl$_2$ at a concentration of 25 mM/ml sample)
optionally a coagulation inhibitor (e.g. activated protein C at a concentration of 1 U/ml)
optionally a fibrin polymerisation inhibitor (e.g. H-Gly-Pro-Arg-Pro-OH AcOH in a concentration of 5 mg/ml sample)

Two or several (or all) of these reagents can be combined in one reagent in order to reduce the number of pipetting steps required. When several components are combined in one reagent it must be evaluated whether the solutions are compatible.

In order to perform the assay procedure a blood or plasma sample is mixed with the reagents and the generation of the conversion product determined. If a chromogenic substrate is applied the reaction can be detected optically at 405 nm (in this case only plasma can normally be analysed). If a fluorogenic substrate is applied platelet poor or platelet rich plasma can be analysed. If a amperogenic or equivalent substrate is applied (i.e. when no optical signal is generated) then blood or plasma can be analysed.

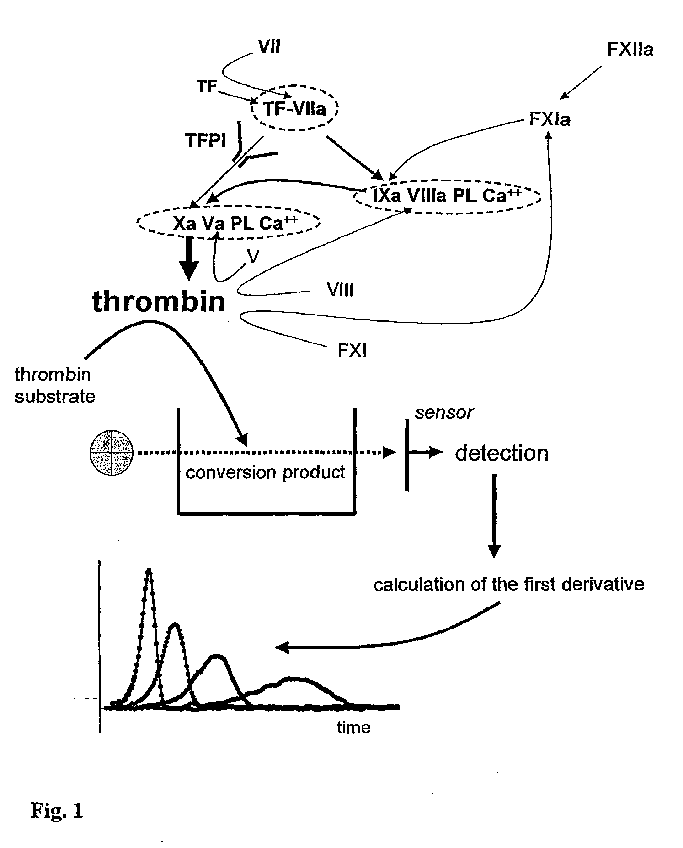

For the analysis of the reaction the conversion of the thrombin substrate is recorded and at least one parameter which depends on the velocity of the conversion of the thrombin substrate is calculated. This is represented in Figure 1 which shows the complex enzymatic interactions which lead to the formation of free thrombin. The initial thrombin formation is detected using a fast thrombin substrate. The conversion of the thrombin substrate is detected using appropriate methods and the first derivative is calculated.

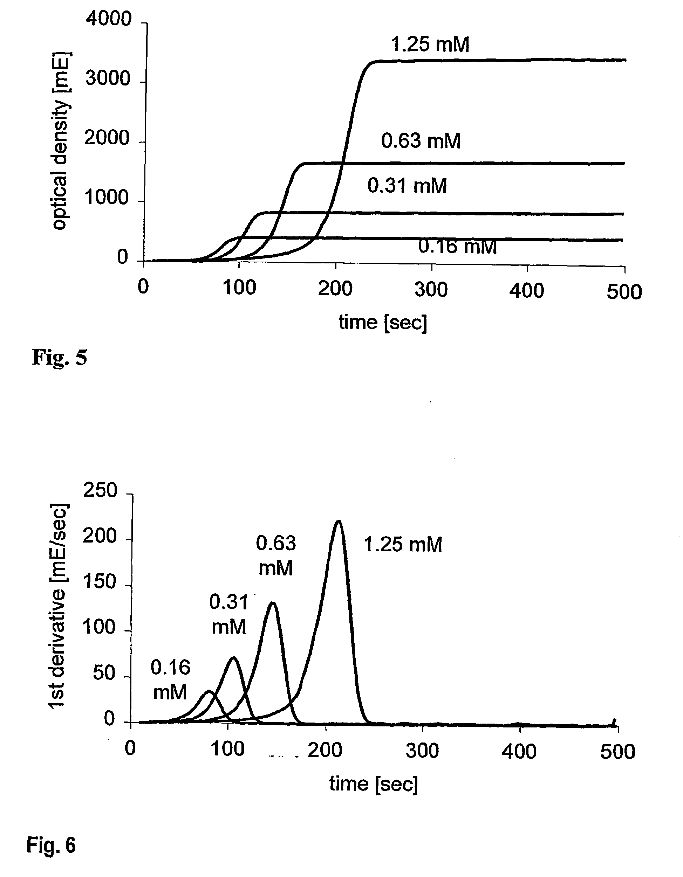

The optical density (OD) of the reagent-sample mixture is typically recorded every 0.5 to 2 seconds. In the experiments which follow it was measured on a standard chromogenic analyser every 2 seconds. The values can be plotted by hand or on a computer to obtain curves as shown in Fig. 5. The change in optical density between successive measurements is then plotted. Although this can be done by hand, a computer program is preferably used. In order to calculate values for the steepness of the curve (the first derivative) the following calculation was performed in Microsoft® Excel®:

```
steepness of the curve at the time point x =
[(OD at time point x + 2 sec.) - (OD at the time point
x - 2 sec)] / 4
```

where x stands for each time point during the analysis (except for the first two and the last two seconds).

The values obtained were plotted to obtain curves of the first derivative as shown in Fig. 6. The peak value of the curves (the maximum of the first derivative) is taken as a measure dependent on the coagulation or thrombin-forming potential for the sample in question.

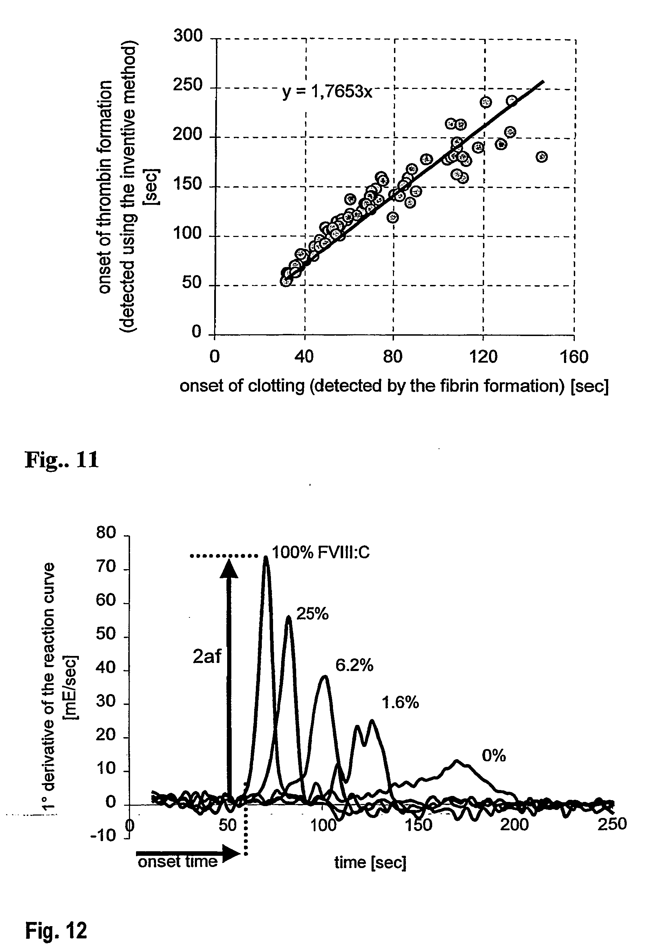

If desired, a computer program may be used to select more precisely the upward points of inflection of the primary curves of Figure 5 (which correspond to the peak values in Figure 6.). An absolute value for a sample relative to the thrombin activity of normal blood can be obtained by comparing the peak of the sample curve with that of plasma from normal blood measured under the same conditions, as described with reference to Fig. 12 below.

Furthermore, an instrument could be arranged with the necessary software to enable point-of-care measurements (e.g. amperometric) calibrated to indicate the thrombin-forming potential of a blood sample using such peak values of the first derivative. Such instruments exist for conventional assays.

The first derivative of the reaction curve reflects the conversion of the thrombin substrate during the assay. The velocity of the conversion of the thrombin substrate depends on whether thrombin is produced fast or slowly during the reaction. We define the maximum of the first derivative as the "initial thrombin formation" (2af). Using a commercially available calibration plasma the initial thrombin formation (2af) can be converted to a % scale, in which 100% corresponds to a normal blood sample and 0% reflects no thrombin activation at all.

Using the method according to the present invention the thrombin activation can be determined using a fast and inexpensive method, using standard instrumentation. Compared to previous methods the assay is much less time consuming (5-10 minutes, compared to 30 minutes of previous methods), requires a much lower quantity of the thrombin substrate (12.5 nmol substrate, compared to up to 450 nmol required for previously applied methods) and can be fully automated on standard equipment.

Most of the examples for the application of the inventive assay presented use chromogenic substrates for the determination of thrombin activity. However also fluorogenic, amperogenic or other substrates can be used for detecting thrombin activity. Although in the examples the reagents are applied in aqueous solution, modifications of the inventive assay can be applied that apply the reagent in dry form. The blood or plasma sample then dissolves the dry reagents and the reaction is again conducted in the liquid phase.

For this application part of the reagents can also be immobilised on solid surfaces (e.g. latex beads).

The method according to the present invention can be adapted to the desired application by the choice of the substrate (chromogenic / fluorogenic / amperogenic), by the concentration of the substrate, by the addition of activators (Tissue Factor, contact activator) or inhibitors (activated protein C, Tissue Factor pathway inhibitor). It generates a strong optical signal and can be analysed using simple algorithms (first derivative). By the use of a fast chromogenic substrate the assay focuses on the initial thrombin activation phase. In contrast to this previously applied methods, especially the ETP assay by Hemker et al, determine the thrombin activation and thrombin inhibition phase. According to Hemker et al the "man hours" of thrombin are the determining factor for hemostasis, bleeding or thrombosis. Therefore in the ETP method the area under the curve of the thrombin activity is calculated as illustrated in Figure 2. This curve, from the physiological point of view, has two parts: the thrombin formation phase and the thrombin inhibition phase. The velocity of thrombin formation in the first part of the curve provides information on whether thrombin is formed fast (sometimes called "burst kinetics") or slowly. This information, which is not provided by the standard coagulation assays, provides valuable additional information for the assessment of hemostasis. The thrombin inhibition phase as assessed by the ETP *in vitro* is determined mainly by the antithrombin activity and by other endogenous and exogenous anticoagulants in the sample. However in the body the down-regulation of thrombin formation and its inhibition are strongly influenced by endothelial structures (thrombomodulin) and substances released by or attached on the endothelium (e.g. glycosaminoglycans). Thus the thrombin inhibition phase as assessed *in vitro* is of low clinical relevance and obscures the clinical predictivity of the ETP method. In contrast to this the inventive assay, by focusing on the initial thrombin activation phase, provides more clinically relevant information on the thrombin activation than previous methods.

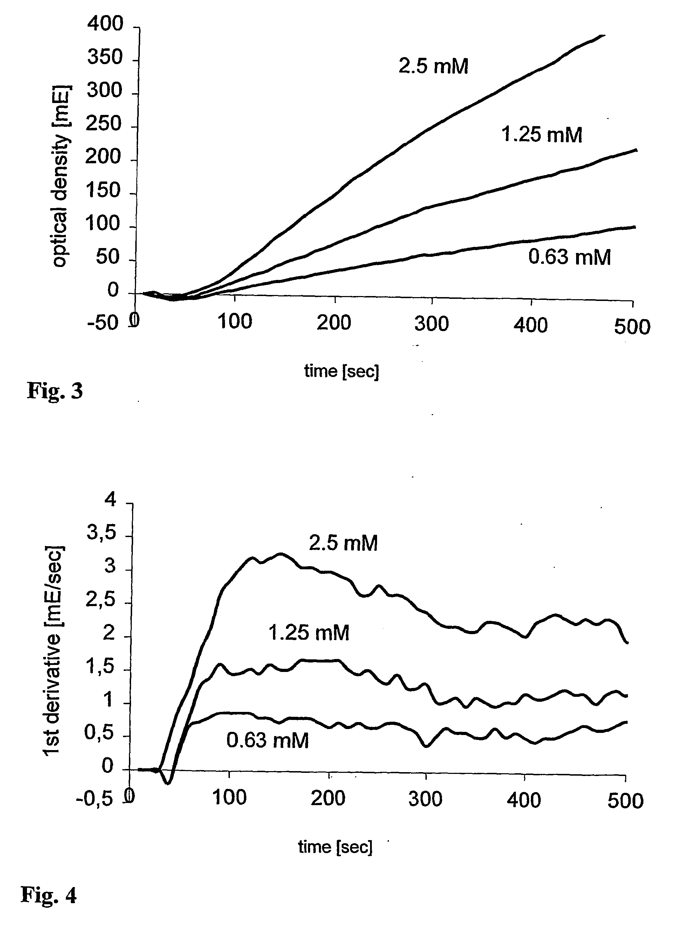

Figure 3 shows the development of the optical density using previously described methods. Thrombin formation was detected using the slow chromogenic substrate H-β-Ala-Gly-Arg-pNA°2AcOH ($K_M$ = 2000 $\mu$M). Fibrin polymerisation was inhibited using the synthetic peptide H-Gly-Pro-Arg-Pro-OH AcOH. Thrombin formation starts after approximately 80 seconds. Figure. 4 shows the first derivative of the reaction curve for the same time periods. The different curves show the effect of varying concentrations of the chromogenic substrate. The rise of the optical density does not stop during the reaction, as the chromogenic substrate is not consumed during the reaction. The chromogenic substrate and the fibrin polymerisation inhibitor are commercially available from Pentapharm Ltd., Basle.

Example 1

For the assessment of the inventive assay the following solutions were prepared:

solution 1 an aqueous solution containing CaCl$_2$ (25 mM), a chromogenic substrate (250 $\mu$M H-D-CHG-Ala-Arg-pNa·2AcOH, Pentapharm, Basle) and a fibrin polymerisation inhibitor (5 mg/ml H-Gly-Pro-Arg-Pro-OH AcOH, Pentapharm, Basle).

solution 2 an aqueous solution containing a contact activator (aluminium silicate 5 g/l, Sigma, St. Louis, USA) and phospholipids (derived from rabbit brain cephalin, 50 $\mu$g/ml, Pentapharm, Basle).

Platelet poor plasma was prepared from venous citrated blood by centrifugation (20 min. at 1500 g).

The procedure was performed as follows:

50 $\mu$l platelet poor plasma was mixed with 50 $\mu$l solution 2 and incubated for 180 seconds. 50 $\mu$l solution 1 was then added and detection of the optical density carried out at 405 nm over 400 seconds.

This procedure is generally followed with specified variations in the (inventive) examples which follow.

Example 2

The procedure was repeated with different substrate concentrations of the substrate H-D-CHG-Ala-Arg-pNa·2AcOH ($K_M$ 15,9 $\mu$M, Pentapharm, Basle)to obtain the graph of Figure 5.

From the reaction curves the first derivative was calculated by determining the rise of optical density over each 4 seconds as already described. The first derivative is plotted in Figure 6.

Figure 5 demonstrates the thrombin formation in the same plasma as in Figures 3 and 4 detected with the method according to the present invention. Using the inventive method a much stronger (20 times stronger) optical signal is generated. The rise of the optical density stops when the substrate is consumed, typically 1-2 min. after the onset of thrombin formation. Figure 5 and Figure 6 show the effect of rising concentrations of the substrate on the detection properties. Rising concentrations of the substrate lead to a rising optical signal. However already the lowest concentrations tested are sufficient for generating a stronger signal than using the previously applied methods. Using the same substrate concentration a more than 100 times stronger signal can be generated when compared to the prior art. According to the inventive assay the optical signal can be enhanced depending on the concentration of the thrombin substrate applied.

Example 3

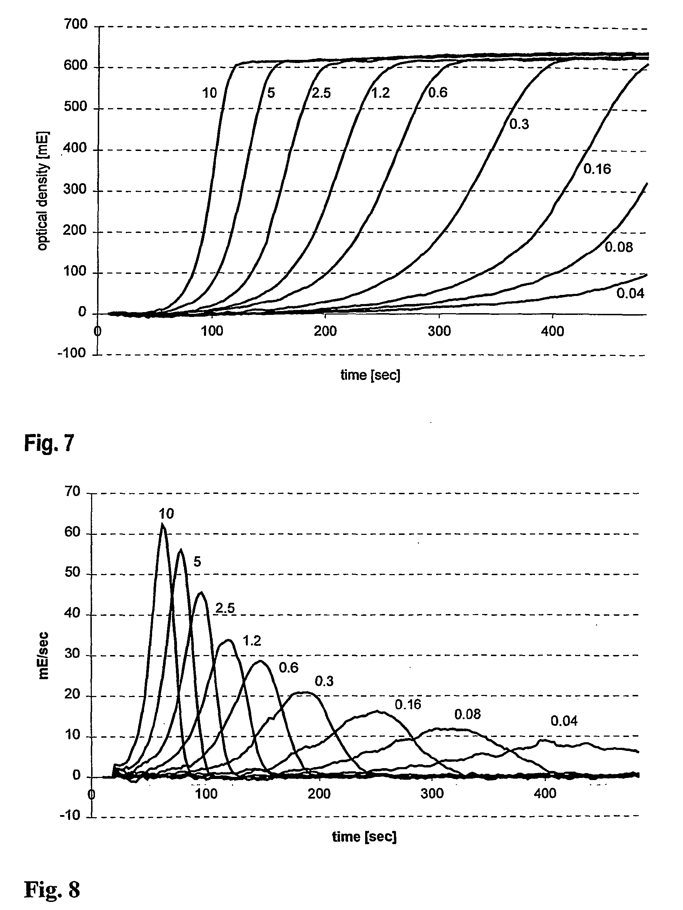

The procedure of Example 1 was repeated using different concentrations of the activator of the plasmatic coagulation system on the assessment of the thrombin formation using the inventive assay. Recombinant Tissue Factor (Instrumentation Laboratory, Kirchheim, Germany) was serially diluted and used as the activator in solution 2. The results of the optical densities and first derivatives are shown respectively in Figure 7 and 8. The numbers in the diagrams show the concentration of recombinant Tissue Factor [ng/ml sample]. Thus the assay can be adapted as required depending on the activation procedure applied.

Example 4 (Comparative)

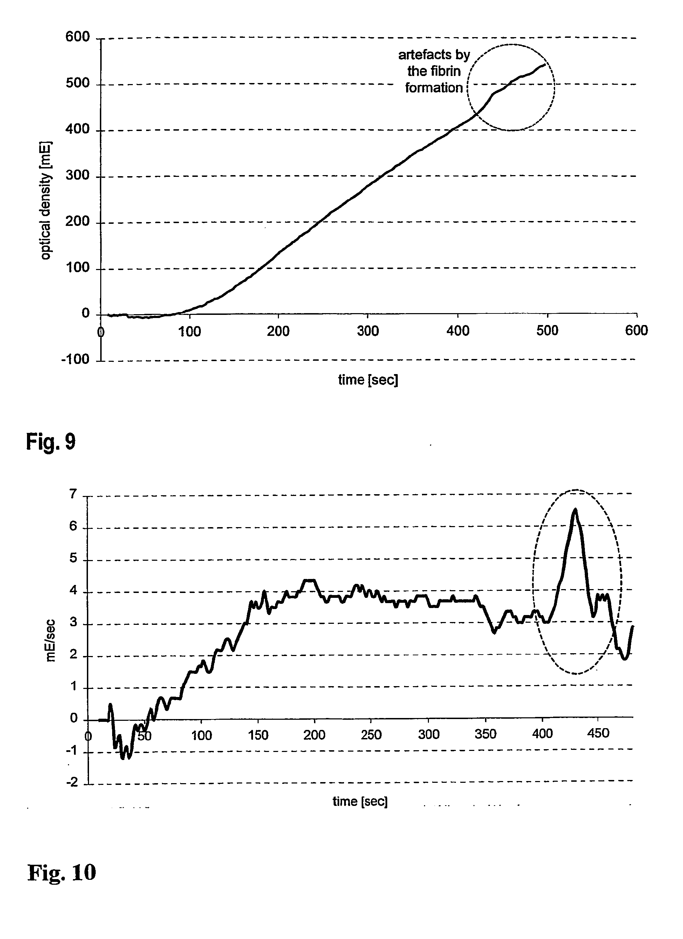

Figure 9 and 10 demonstrate one of the limitations of the previous art. The thrombin formation was assessed using the slow thrombin substrate
H-β-Ala-Gly-Arg-pNA°2AcOH (2.5 mM) and the fibrin polymerisation inhibitor
H-Gly-Pro-Arg-Pro-OH AcOH (5mg/ml). At 400 sec. after the onset of thrombin formation optical anomalies are detected, which are even more apparent in the first derivative (Figure. 10). Most likely these are due to late fibrin gelation phenomena, which occurred in spite of the use of the fibrin polymerisation inhibitor. Fibrinogen is an acute phase protein and is significantly enhanced in many states of disease. Apparently the use of the fibrin polymerisation inhibitor does not completely abolish fibrin gelation in samples with enhanced fibrinogen concentration.
In the inventive method the optical signal is much stronger, so optical anomalies disturb the reaction in a much lower extend. In addition using the inventive method the detection of the thrombin formation ends typically within 1-2 minutes (when the thrombin substrate is consumed), so late fibrin gelation phenomena (as seen in Figures. 9 and 10) do not affect the measurement.

Example 5

Figure 11 compares the detection of the onset of coagulation by the Fibrin Formation method and by the inventive method. For this experiment 80 plasma samples from healthy volunteers and hemophilia A patients were clotted by the addition of a contact phase activator (aluminium silicate, Sigma, St. Louis, USA), phospholipids (rabbit brain cephalin, Pentapharm, Basle) and $CaCl_2$ (Sigma, St. Louis, USA). This procedure is called "Activated Partial Thromboplastin time" (aPTT). The time from the start of the procedure till clotting is detected is the aPTT. The inventive assay was performed using the identical activation procedure as the aPTT. The difference between the two procedures was the addition of the fast chromogenic substrate
(H-D-CHG-Ala-Arg-pNa·2AcOH, 250 μmol/l, Pentapharm) and the fibrin polymerisation inhibitor H-Gly-Pro-Arg-Pro-OH AcOH (5mg/ml, Pentapharm) in the inventive assay.
Figure 11 shows that the addition of the chromogenic substrate delayed the onset of the thrombin formation (as this was proposed by Hemker). However unexpectedly (and in contrast to the publications by Hemker et al) the inhibition of coagulation by the addition of the fast chromogenic substrate resulted in a highly systematic effect, which does not interfere with the correct assessment of hemostasis. The correlation of the two methods was excellent (correlation coefficient 0.95).

Example 6

Figure 12 demonstrates the effect of decreasing activities of the coagulation factor FVIII on the assay according to the present invention. A commercially available calibration plasma (produced using a plasma pool from healthy volunteers, Instrumentation Laboratory, Kirchheim, Germany) was used for calibrating the results. The first derivative as a percentage of 'normal' is defined as the initial thrombin formation (2af), which is set for the calibration plasma as 100% .
The plasma samples were prepared by serially diluting the calibration plasma using FVIII deficient plasma (a plasma which has been depleted from factor VIII, commercially available from Dade-Behring, Marburg, Germany). Figure. 12 shows that the thrombin formation as assessed by the inventive assay is highly dependent on the FVIII activity in the sample. FVIII is the coagulation factor, whose concentration or activity is decreased in the hemophilia A patients. This deficiency results in a decreased thrombin formation and therefore in a severe bleeding tendency in these patients.

Example 7

For the assessment of the effect of factor VIII supplementation on the initial thrombin formation the following solutions are prepared:

solution 1 an aqueous solution containing CaCl$_2$ (25 mM), a chromogenic substrate (250 $\mu$M H-D-CHG-Ala-Arg-pNa·2AcOH, Pentapharm, Basle) and a fibrin polymerisation inhibitor (5 mg H-Gly-Pro-Arg-Pro-OH AcOH / ml, Pentapharm, Basle).

solution 2 an aqueous solution containing a contact activator (aluminium silicate 5 g/l, Sigma, St. Louis, USA) and phospholipids (derived from rabbit brain cephalin, 50 $\mu$g/ml, Pentapharm, Basle).

solution 3 an aqueous solution containing recombinant Tissue Factor (Baxter, Vienna, Austria) in a concentration of 1 U/ml.

Platelet poor plasma is prepared from venous citrated blood by centrifugation (20 min at 1500 g).
The procedure is performed as follows:

50 $\mu$l platelet poor plasma was mixed with 50 $\mu$l solution 2 and 50 $\mu$l solution 3 and incubated for 600 seconds. 50 $\mu$l solution 1 was added and the optical density detected at 405 nm over 400 seconds.

During the incubation period the added FVIII from solution 3 can be inhibited by anti-FVIII-antibodies in the patient sample. The effect of FVIII supplementation and FVIII inhibition on the thrombin formation is assessed by this method.

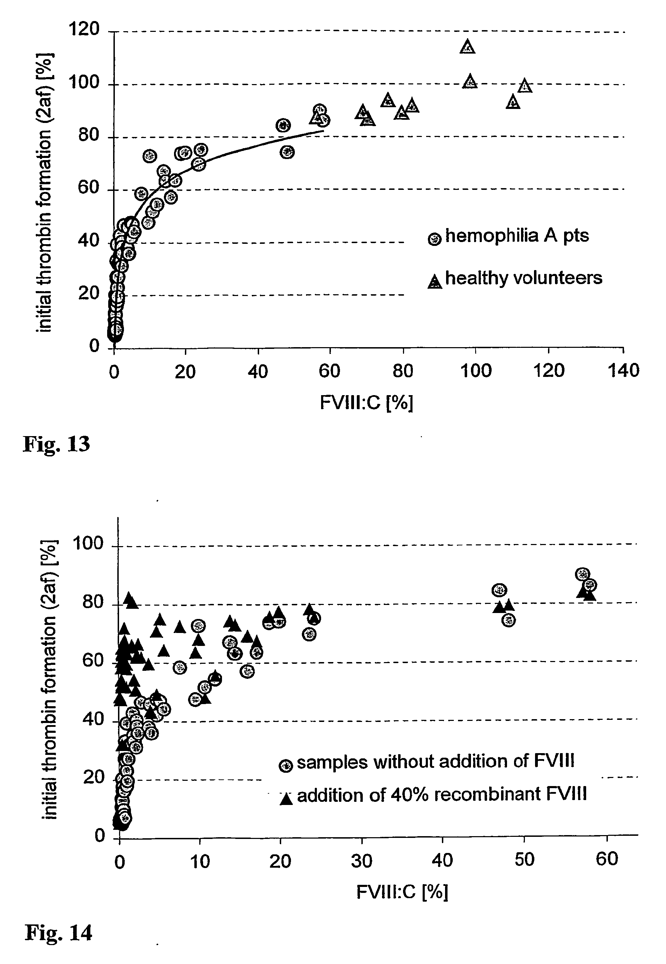

Figure 13 shows the correlation of factor VIII activity and the initial thrombin formation (2af) in hemophilia A patients and healthy volunteers. The initial thrombin formation (2af) was given as '% of normal' using calibration from a normal plasma pool (which was set as 100%).
The results show a very good, non-linear correlation of the two parameters. For plasma samples with a factor VIII activity below 20% (of normal) there is a FVIII-dependent decrease of the thrombin formation as assessed by the inventive assay.
Figure 14 demonstrates the effect of the addition of FVIII (recombinant, Baxter, Vienna, Austria) to hemophilia A patients on the initial thrombin formation. The initial thrombin formation was again given as '% of normal' using calibration from a normal plasma pool (which was set as 100%).
The addition of FVIII corrected the FVIII deficiency in the samples and resulted in a significant increase of the initial thrombin formation determined by the inventive assay.

Example 8 a - c

Figure 15 and 16 demonstrate different experiments which show the correlation of the initial thrombin formation by the inventive method and the Prothrombin Time method.
For the assessment of the inventive assay the following aqueous solutions were prepared:

Solution 1a an aqueous solution prepared by combining CaCl$_2$ (25 mM), a chromogenic substrate (250 $\mu$M H-D-CHG-Ala-Arg-pNa·2AcOH, Pentapharm, Basle), a fibrin polymerisation inhibitor (5 mg/ml H-Gly-Pro-Arg-Pro-OH AcOH, Pentapharm, Basle) and recombinant Tissue Factor (200 ng/ml, Instrumentation Laboratory, Kirchheim, Germany).

Solution 1b an aqueous solution prepared by combining CaCl$_2$ (25 mM), a chromogenic substrate (250 $\mu$M H-D-CHG-Ala-Arg-pNa·2AcOH), a fibrin polymerisation inhibitor (5 mg H-Gly-Pro-Arg-Pro-OH AcOH / ml) and recombinant Tissue Factor (20 ng/ml) in aqueous solution.

Solution 1c (reduced Tissue Factor content) an aqueous solution prepared by combining CaCl$_2$ (25 mM), a chromogenic substrate (250 $\mu$M
H-D-CHG-Ala-Arg-pNa-2AcOH), a fibrin polymerisation inhibitor (5 mg
H-Gly-Pro-Arg-Pro-OH AcOH / ml) and recombinant Tissue Factor (2 ng/ml) in aqueous solution.

Solution 2 an aqueous solution prepared by combining phospholipids (derived from rabbit brain cephalin, 50 $\mu$g/ml, Pentapharm, Basle) and HEPES buffer (pH 7.4, 50 mM, Sigma, St. Louis) in aqueous solution.

Platelet poor plasma was prepared from venous citrated blood by centrifugation (20 min. at 1500 g).
The procedures were performed as follows:

Example 8a:

50 $\mu$l platelet poor plasma was mixed with 50 $\mu$l solution 2 and incubated for 180 seconds. 50 $\mu$l solution 1a was added and the optical density detected at 405 nm over 400 seconds.

Example 8b:

50 $\mu$l platelet poor plasma was mixed with 50 $\mu$l solution 2 and incubated for 180 seconds. 50 $\mu$l solution 1b was added and the optical density detected at 405 nm over 400 seconds.

Example 8c:

50 $\mu$l platelet poor plasma was mixed with 50 $\mu$l solution 2 and incubated for 180 seconds. 50 $\mu$l solution 1c was added and the optical density detected at 405 nm over 400 seconds.

From the reaction curve the first derivative was calculated by determining the rise of optical density over each 4 seconds. The maximum of the first derivative was defined as the initial thrombin activity (2af). A commercially available calibration plasma (produced using a plasma pool from healthy volunteers, commercially available from Instrumentation Laboratory, Kirchheim, Germany) was used for calibrating the results. The initial thrombin activity of the calibration plasma was set as 100% (of normal).

Citrated plasma from 50 patients with normal or decreased coagulation factor concentrations were assayed. In addition to the inventive method the standard assay Prothrombin Time was performed (using the reagent Recombiplastin® by Instrumentation Laboratory, Kirchheim, Germany). The Prothrombin Time reflects the coagulation factor concentration of the sample, and is performed by adding to the a sample a reagent containing Tissue Factor in a very high concentration, phospholipids and CaCl$_2$. The Prothrombin Time was performed as follows:

50 $\mu$l plasma was mixed with 100 $\mu$l Recombiplastin® and the clotting time detected at 405 nm.

Figure 15 shows the correlation of the initial thrombin activity determined using the inventive assay to the Prothrombin Time. Using the method according to Example 8a, a higher thrombin formation was detected when compared to 8b and 8c (the latter method resulted in the weakest thrombin formation). This is due to the decreasing amount of Tissue Factor which was used in the examples. This also corresponds to the results of the experiment shown in Figures 7 and 8.

In Figure 16 the thrombin activity assessed was transformed into % of normal (by calibrating it against a normal plasma pool) and shown versus the Prothrombin Time (in % of normal). The initial thrombin activity depends on the coagulation factor activity in the sample (as shown by the significant correlation of the initial thrombin formation versus the Prothrombin Time). However considerable variation is also seen, especially when the lowest Tissue Factor activation is applied (Example 8c).

Thus information which is not provided by the Prothrombin Time method is determined by the assessment of the initial thrombin formation using the inventive assay.

Example 9:

For the assessment of the interaction of plasmatic and cellular components of coagulation the following solutions are prepared:

solution 1 An aqueous solution containing CaCl$_2$ (25 mM) and a fluorogenic thrombin substrate (H-D-Cha-Ala-Arg-AMC 2 AcOH, 25 $\mu$M, Pentapharm, Basle).
AMC: 7-Amino-4-methylcoumarin

solution 2 A second solution is prepared by combining a contact activator (aluminium silicate 5 g/l, Sigma, St. Louis, USA) and phospholipids (derived from rabbit brain cephalin, 50 $\mu$g/ml, Pentapharm, Basle) in aqueous solution.

Platelet rich plasma is prepared from venous citrated blood by centrifugation (20 min. at 500 g).

The procedure is performed as follows:

50 $\mu$l platelet poor plasma is mixed with 50 $\mu$l solution 2 and incubated for 180 seconds. 50 $\mu$l solution 1 is added and the light emission at about 440 nm detected (excitation wavelength about 342 nm).

Due to the fluorogenic detection no means for inhibiting fibrin gelation are required. Due to the use of platelet rich plasma in this example the effect of platelet inhibition on thrombin formation can be assessed.

Example 10:

For the assessment of the effect of disorders of the protein C system on the initial thrombin formation the following solutions are prepared:

solution 1 An aqueous solution containing $CaCl_2$ (25 mM), a chromogenic substrate (250 $\mu$M H-D-CHG-Ala-Arg-pNa·2AcOH, Pentapharm, Basle) and a fibrin polymerisation inhibitor (5 mg/ml H-Gly-Pro-Arg-Pro-OH AcOH, Pentapharm, Basle).

solution 2 A second solution is prepared by combining a contact activator (aluminium silicate 5 g/l, Sigma, St. Louis, USA) and phospholipids (derived from rabbit brain cephalin, 50 $\mu$g/ml, Pentapharm, Basle) in aqueous solution.

solution 3 A third solution is prepared containing activated protein C (Enzyme Research, South Bend, IN, USA) in a concentration of 1 U/ml.

Platelet poor plasma is prepared from venous citrated blood by centrifugation (20 min. at 1500 g).
The procedure is performed as follows:

50 $\mu$l platelet poor plasma is mixed with 50 $\mu$l solution 2 and 50 $\mu$l solution 3 and incubated for 180 seconds. 50 $\mu$l solution 1 is added and the optical density detected at 405 nm over 400 seconds.

The activated Protein C present in solution 3 inactivates FVa from the sample and reduces the thrombin formation. In patients with inherited (e.g. carriers of the factor V Leiden mutation) or acquired disorders of the activated Protein C system (e.g. due to oral contraception) the inhibition of thrombin activation is decreased when compared to normal subjects.

**Claims**

1. A method of assessing the coagulation potential of a sample of blood or plasma comprising:

   a) mixing a blood or plasma sample with at least one activator of the blood coagulation system, a thrombin substrate, and a substance interfering with fibrin gelation, the sample and activator mixture having been incubated if necessary,
   b) incrementally or continuously determining the release of the conversion product of the thrombin substrate, and
   c) calculating at least one value indicative of the coagulation potential,

   wherein:

   i.) the quantity of substrate is sufficiently small to be completely consumed by a specified quantity of normal blood or plasma,
   the amount and kinetic properties of the substrate are selected such that said consumption takes place within a range from 5 to 600 seconds,
   the said value calculated is dependent on the velocity of consumption of the substrate.

2. A method according to claim 1 in which the substance which interferes with the fibrin gelation is a fibrin polymerisation inhibitor.

3. A method according to claim 2 wherein the fibrin polymerisation inhibitor is H-Gly-Pro-Arg-Pro-OH AcOH.

4. A method according to any preceding claim wherein the said value is the maximum rate of consumption of the substrate, being dependent upon the initial thrombin activity.

5. A method according to any preceding claim wherein the maximum rate of consumption of substrate of a test sample is compared to a value under the same conditions for a standard blood or plasma sample to provide a measure of the thrombin activity of the test sample.

**6.** A method according to any preceding claim wherein the thrombin substrate has a $K_M$ of 200 $\mu$M or less.

**7.** A method according to any preceding claim wherein the thrombin substrate has a $K_M$ of 100 $\mu$M or less.

**8.** A method according to any preceding claim wherein the thrombin substrate has a $K_M$ of 50 $\mu$M or less.

**9.** A method according to any preceding claim for assessing plasma, wherein the thrombin substrate is a chromogenic substrate.

**10.** A method according to claim 9 wherein the chromogenic substrate is selected from:

H-D-CHG-Ala-Arg-pNa·2AcOH,
H-D-Phe-Pip-Arg-pNA·2HCl,
Boc-Asp(OBzl)-Pro-Arg-NH-Mec,
pyroGlu-Pro-Arg-pNA·HCl and
Bz-Phe-Val-Arg-pNA.

**11.** A method according to either of claims 7 or 8 wherein the conversion product is detected optically at 405 nm.

**12.** A method according to any of claims 1 to 8 wherein the thrombin substrate is a fluorogenic or amperogenic substrate.

**13.** A method according to any preceding claim wherein at least one coagulation inhibitor is used in step (a).

**14.** A method according to claim 13, wherein the coagulation inhibitor is selected from one or more of:

Protein C activator,
Tissue Factor Pathway Inhibitor (or equivalent substances),
inactivated factor VIIa (or equivalent substances),
Activated Protein C (or equivalent substances),
Prostacyclin or other platelet inhibitors,
FVIII inhibitor (or equivalent substances),
FIX inhibitor (or equivalent substances),
heparins and/or heparinoids,
direct thrombin inhibitors,
direct Factor Xa inhibitors and
aprotinin or other serine protease inhibitors.

**15.** A method according to any preceding claim further comprising at least one coagulation accelerant in step (a).

**16.** A method according to claim 15, wherein the coagulation accelerant is selected from one or more of phospholipids of natural or synthetic origin or analogous substances, calcium chloride or other sources of divalent cations.

**17.** A method according to any preceding claim wherein the activator or activators of the coagulation system are selected from one or more of:

Tissue Factor or analogous substances,
activators of factor X ,
activators of factor V ,
prothrombin activators ,
kaolin or other contact phase activators,
activated coagulation factors or analogous substances and
collagen or other platelet activators.

**18.** A method according to claim 17 including at least on snake venom activator.

**19.** A method according to any preceding claim utilising a solution of said substrate in a concentration less than or equal to 1000$\mu$M based on a volume ratio of 1:1 with plasma.

**20.** A method according to any preceding claim utilising a solution of said substrate in a concentration less than or equal to 500$\mu$M based on a volume ratio of 1:1 with plasma.

**21.** A method according to any preceding claim utilising a solution of said substrate in a concentration less than or equal to 250$\mu$M based on a volume ratio of 1:1 with plasma.

**Patentansprüche**

**1.** Verfahren zur Bestimmung des Koagulationspotentials einer Blut- oder Plasmaprobe umfassend:

a) Mischen einer Blut- oder Plasmaprobe mit mindestens einem Aktivator des Blutkoagulationssystems, einem Thrombinsubstrat und einer mit der Fibrin-Gelierung interferierenden Substanz, wobei die Mischung der Probe und des Aktivators gegebenenfalls inkubiert worden ist,
b) schrittweises oder kontinuierliches Bestimmen der Freisetzung des Umsetzungsprodukts des Thrombinsubstrats, und
c) Berechnen von mindestens einem indikativen Wert des Koagulationspotentials,

wobei:

i.) die Substratmenge genügend klein ist, um vollständig von einer bestimmten Menge normalem Blut oder Plasma verbraucht zu werden,
die Menge und die kinetischen Eigenschaften des Substrats derart ausgewählt sind, dass der besagte Verbrauch in einem Bereich von 5 bis 600 Sekunden stattfindet,
der besagte berechnete Wert von der Geschwindigkeit des Verbrauchs des Substrats abhängig ist.

**2.** Verfahren nach Anspruch 1, wobei die Substanz, welche mit der Fibrin-Gelierung interferiert, ein Fibrin-Polymerisationsinhibitor ist.

**3.** Verfahren nach Anspruch 2, wobei der Fibrin-Polymerisationsinhibitor H-Gly-Pro-Arg-Pro-OH AcOH ist.

**4.** Verfahren nach einem der vorangehenden Ansprüche, wobei der besagte Wert die maximale Verbrauchsrate des Substrats ist, welche von der initialen Thrombinaktivität abhängt.

**5.** Verfahren nach einem der vorangehenden Ansprüche, wobei die maximale Verbrauchsrate des Substrats einer Testprobe mit einem Wert verglichen wird, welcher unter gleichen Bedingungen für eine standardmässige Blut- oder Plasmaprobe erhalten wurde, um einen Messwert der Thrombinaktivität der Testprobe zur Verfügung zu stellen.

**6.** Verfahren nach einem der vorangehenden Ansprüche, wobei das Thrombinsubstrat einen $K_M$ von 200$\mu$M oder weniger aufweist.

**7.** Verfahren nach einem der vorangehenden Ansprüche, wobei das Thrombinsubstrat einen $K_M$ von 100$\mu$M oder weniger aufweist.

**8.** Verfahren nach einem der vorangehenden Ansprüche, wobei das Thrombinsubstrat einen $K_M$ von 50$\mu$M oder weniger aufweist.

**9.** Verfahren nach einem der vorangehenden Ansprüche für die Plasmabestimmung, wobei das Thrombinsubstrat ein chromogenes Substrat ist.

**10.** Verfahren nach Anspruch 9, wobei das chromogene Substrat ausgewählt ist von:

H-D-CHG-Ala-Arg-pNa - 2AcOH,
H-D-Phe-Pip-Arg-pNA·2HCl,
Boc-Asp(OBzl)-Pro-Arg-NH-Mec,
pyroGlu-Pro-Arg-pNA·HCl und
Bz-Phe-Val-Arg-pNA.

**11.** Verfahren nach einem der Ansprüche 7 oder 8, wobei das Umsetzungsprodukt optisch bei 405 nm erfasst wird.

**12.** Verfahren nach einem der Ansprüche 1 bis 8, wobei das Thrombinsubstrat ein fluorogenes oder amperogenes Substrat ist.

**13.** Verfahren nach einem der vorangehenden Ansprüche, wobei mindestens ein Koagulationsinhibitor in Stufe (a) verwendet wird.

**14.** Verfahren nach Anspruch 13, wobei der Koagulationsinhibitor ausgewählt ist von einem oder mehreren von:

Protein C Aktivator,
TFPI Gewebefaktor-Inhibitor (oder äquivalente Substanzen),
inaktivierter Faktor VIIa (oder äquivalente Substanzen),
aktiviertes Protein C (oder äquivalente Substanzen),
Prostazyclin oder andere Blutplättcheninhibitoren,
FVIII Inhibitor (oder äquivalente Substanzen),
FIX Inhibitor (oder äquivalente Substanzen),
Heparine und/oder Heparinoide,
direkte Thrombininhibitoren,
direkte Inhibitoren des Faktors Xa und
Aprotinin oder andere Serin-Protease-Inhibitoren.

**15.** Verfahren nach einem der vorangehenden Ansprüche, welches des Weiteren mindestens einen Koagulationsbeschleuniger in Stufe (a) umfasst.

**16.** Verfahren nach Anspruch 15, wobei der Koagulationsbeschleuniger ausgewählt ist von einem oder mehreren der Phospholipide natürlicher oder synthetischer Herkunft oder analogen Substanzen, Kalziumchlorid oder anderen Quellen von bivalenten Kationen.

**17.** Verfahren nach einem der vorangehenden Ansprüche, wobei der Aktivator oder die Aktivatoren des Koagulationssystems ausgewählt sind von einem oder mehreren von:

Gewebefaktor oder analoge Substanzen,
Aktivatoren des Faktors X,
Aktivatoren des Faktors V,
Prothrombinaktivatoren,
Kaolin oder andere Kontaktphasen-Aktivatoren,
aktivierte Koagulationsfaktoren oder analoge Substanzen und
Kollagen oder andere Blutplättchenaktivatoren.

**18.** Verfahren nach Anspruch 17, welches mindestens einen Schlangengift-Aktivator umfasst.

**19.** Verfahren nach einem der vorangehenden Ansprüche, welches eine Lösung des besagten Substrats in einer Konzentration von weniger als oder gleich 1000 µM basierend auf einem Volumenverhältnis von 1 : 1 zum Plasma verwendet.

**20.** Verfahren nach einem der vorangehenden Ansprüche, welches eine Lösung des besagten Substrats in einer Konzentration von weniger als oder gleich 500 µM basierend auf einem Volumenverhältnis von 1 : 1 zum Plasma verwendet.

**21.** Verfahren nach einem der vorangehenden Ansprüche, welches eine Lösung des besagten Substrats in einer Konzentration von weniger als oder gleich 250 µM basierend auf einem Volumenverhältnis von 1 : 1 zum Plasma verwendet.

**Revendications**

**1.** Méthode de dosage du potentiel de coagulation d'un échantillon de sang ou de plasma comprenant :

a) mélanger un échantillon de sang ou de plasma avec au moins un activateur du système de la coagulation sanguine, un substrat de la thrombine et une substance interférant avec la gélification de la fibrine, le mélange de l'échantillon et de l'activateur ayant été optionnellement incubé,

b) déterminer de manière incrémentale ou continue la libération du produit de conversion du substrat de la thrombine, et

c) calculer au moins une valeur indicative du potentiel de coagulation

où :

i.) la quantité de substrat est suffisamment petite pour être complètement absorbée par une quantité spécifique de sang ou de plasma normal,

la quantité et les propriétés cinétiques du substrat sont choisies de telle façon que ladite absorption a lieu dans un temps compris entre 5 et 600 secondes,

ladite valeur calculée dépend de la vélocité de l'absorption du substrat.

2. Méthode selon la revendication 1, où la substance qui interfère avec la gélification de la fibrine est un inhibiteur de la polymérisation de la fibrine.

3. Méthode selon la revendication 2, où l'inhibiteur de la polymérisation de la fibrine est H-Gly-Pro-Arg-Pro-OH AcOH.

4. Méthode selon l'une quelconque des revendications précédentes, où ladite valeur est le taux maximum d'absorption du substrat qui dépend de l'activité de la thrombine initiale.

5. Méthode selon l'une quelconque des revendications précédentes, où le taux maximum d'absorption du substrat d'un échantillon d'essai est comparé à une valeur obtenue pour un échantillon de sang ou de plasma standard sous les mêmes conditions pour fournir une mesure de l'activité de la thrombine de l'échantillon d'essai.

6. Méthode selon l'une quelconque des revendications précédentes, où le substrat de la thrombine a un $K_M$ de 200$\mu$M ou moins.

7. Méthode selon l'une quelconque des revendications précédentes, où le substrat de la thrombine a un $K_M$ de 100$\mu$M ou moins.

8. Méthode selon l'une quelconque des revendications précédentes, où le substrat de la thrombine a un $K_M$ de 50$\mu$M ou moins.

9. Méthode selon l'une quelconque des revendications précédentes pour le dosage de plasma, où le substrat de la thrombine est un substrat chromogène.

10. Méthode selon la revendication 9, où le substrat chromogène est choisi parmi :

H-D-CHG-Ala-Arg-pNa · 2AcOH,
H-D-Phe-Pip-Arg-pNA · 2HCl,
Boc-Asp(OBzl)-Pro-Arg-NH-Mec,
pyroGlu-Pro-Arg-pNA · HCl et
Bz-Phe-Val-Arg-pNA.

11. Méthode selon l'une des revendications 7 ou 8, où le produit de conversion est détecté de manière optique à 405 nm.

12. Méthode selon l'une quelconque des revendications 1 à 8, où le substrat de la thrombine est un substrat fluorogène ou ampérogène.

13. Méthode selon l'une quelconque des revendications précédentes, où au moins un inhibiteur de la coagulation est utilisé dans l'étape (a).

14. Méthode selon la revendication 13, où l'inhibiteur de la coagulation est choisi parmi un ou plusieurs de :

activateur de la protéine C,
TFPI inhibiteur de la voie du facteur tissulaire (ou des substances équivalentes),
facteur VIIa inactivé (ou des substances équivalentes),
protéine C activée (ou des substances équivalentes),
prostacycline ou d'autres inhibiteurs des plaquettes,
inhibiteur FVIII (ou des substances équivalentes),
inhibiteur FIX (ou des substances équivalentes),
héparines et/ou héparinoïdes,
inhibiteurs de la thrombine directs,
inhibiteurs du facteur Xa directs et
aprotinine ou d'autres inhibiteurs de la sérine protéase.

15. Méthode selon l'une quelconque des revendications précédentes, comprenant en plus au moins un accélérateur de coagulation dans l'étape (a).

16. Méthode selon la revendication 15, où l'accélérateur de coagulation est choisi parmi un ou plusieurs des phospholipides d'origine naturelle ou synthétique ou des substances analogues, de chlorure de calcium ou d'autres sources de cations divalents.

17. Méthode selon l'une quelconque des revendications précédentes, où l'activateur ou les activateurs du système de coagulation sont choisis parmi un ou plusieurs de :

facteur tissulaire ou des substances analogues,
activateurs du facteur X,
activateurs du facteur V,
activateurs de la prothrombine,
kaolin ou d'autres activateurs de la phase de contact,
des facteurs de la coagulation activés ou des substances analogues et
collagène ou d'autres activateurs des plaquettes.

18. Méthode selon la revendication 17 comprenant au moins un activateur de venin de serpent.

19. Méthode selon l'une quelconque des revendications précédentes, utilisant une solution du substrat en concentration inférieure ou égale à 1000 $\mu$M basée sur une proportion de volume de 1 : 1 par rapport au plasma.

20. Méthode selon l'une quelconque des revendications précédentes, utilisant une solution du substrat en concentration inférieure ou égale à 500 $\mu$M basée sur une proportion de volume de 1 : 1 par rapport au plasma.

21. Méthode selon l'une quelconque des revendications précédentes, où utilisant une solution du substrat en concentration inférieure ou égale à 250 $\mu$M basée sur une proportion de volume de 1 : 1 par rapport au plasma.

Fig. 1

Area under the curve ➡
„endogenous thrombin potential" (ETP)

**Fig. 2**

**Fig. 3**

**Fig. 4**

**Fig. 5**

**Fig. 6**

Fig. 7

Fig. 8

**Fig. 9**

**Fig. 10**

**Fig.. 11**

**Fig. 12**

**Fig. 13**

**Fig. 14**

**Fig. 15**

**Fig. 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9939212 A
- EP O420332 A, Hemker
- DE 19904674 A
- EP 0229234 A

- US 5192689 A, Hemker
- US 4784944 A, Kolde
- US 4289498 A, Baughman

### Non-patent literature cited in the description

- **Rijkers et al.** Design and Synthesis of Thrombin Substrates with Modified Kinetic Parameters. *Thrombosis Research,* 1995, vol. 79 (5/6), 491-499
- **Lottenberg et al.** The Action of Thrombin On Peptide P-Nitroanilide Substrates: Hydrolysis of Tos-Gly-Pro-Arg-Pna and D-Phe-Pip-Arg-Pna by Human Alpha and Gamma and Bovine Alpha and Beta-Thrombins. *Thrombosis Research,* 1982, vol. 28 (1), 313-332
- **Baughman et al.** Thrombin Activation Rate Constant 1 Stage Chromogenic Assay For the Extrinsic System. *Thrombosis Research,* 1982, vol. 26 (1), 1-12
- **De Peuriot et al.** Electrochemical Activity Determination of Trypsin-Like Enzymes 4. Coupled Electrochemical and Spectrophotometric Assay of Thrombin Using the Same D Phenylalanyl Pipecolyl Arginine 4 Methoxy-Beta Naphthylamide Di Hydro Chloride S-2421 Substrates. *Thrombosis Research,* 1981, vol. 22 (3), 303-308
- **Hemker H C ; Beguin S.** Phenotyping the clotting system. *Thromb Haemost,* 2000, vol. 84, 747-51
- **Mann KG.** Biochemistry and physiology of blood coagulation. *Thromb Haemost,* August 1999, vol. 82 (2), 165-74
- **Xi M ; Beguin S ; Hemker H C.** The relative importance of the factors II, VII, IX and X for the prothrombinase activity in plasma of orally anticoagulated patients. *Thromb Haemost,* 1989, vol. 62, 788-91
- **Rosing J ; Hemker HC ; Tans G.** Molecular biology and pathophysiology of APC resistance: current insights and clinical implications. *Semin Thromb Hemost.,* 1998, vol. 24 (4), 329-35
- **Beguin S ; Keularts I.** On the coagulation of platelet-rich plasma. Physiological mechanism and pharmacological consequences. *Haemostasis,* 1999, vol. 29, 50-7

- **Hemker H C ; Wielders S ; Kessels H ; Beguin S.** Continuous registration of thrombin generation in plasma, its use for the determination of the thrombin potential. *Thromb Haemost,* 1993, vol. 70, 617-24
- **Hemker H C ; Beguin S.** Thrombin generation in plasma: its assessment via the endogenous thrombin potential. *Thromb Haemost,* 1995, vol. 74, 134-8
- **Rijkers D T ; Hemker H C ; Tesser G I.** Synthesis of peptide p-nitroanilides mimicking fibrinogen- and hirudin-binding to thrombin. Design of slow reacting thrombin substrates. *Int J Pept Protein Res,* 1996, vol. 48, 182-93
- **Wielders S ; Mukherjee M ; Michiels J ; Rijkers D T ; Cambus J P ; Knebel R W ; Kakkar V ; Hemker H C ; Beguin S.** The routine determination of the endogenous thrombin potential, first results in different forms of hyper- and hypocoagulability. *Thromb Haemost,* 1997, vol. 77, 629-36
- **Kumar R ; Beguin S ; Hemker H C.** The influence of fibrinogen and fibrin on thrombin generation is an evidence for feedback activation of the clotting system by clot bound thrombin. *Thromb Haemost,* 1994, vol. 72, 713-21
- **Prasa D ; Svendsen L ; Sturzebecher J.** Inhibition of thrombin generation in plasma by inhibitors of factor Xa. *Thromb Haemost,* October 1997, vol. 78 (4), 1215-20
- **Prasa D ; Svendsen L ; Sturzebecher J.** The ability of thrombin inhibitors to reduce the thrombin activity generated in plasma on extrinsic and intrinsic activation. *Thromb Haemost,* 1997, vol. 77 (3), 498-503
- **Hemker H C ; Giesen P L ; Ramjee M ; Wagenvoord R ; Beguin S.** The thrombogram: monitoring thrombin generation in platelet-rich plasma. *Thromb Haemost,* 2000, vol. 83, 589-91

- **Keularts I M ; Hamulyak K ; Hemker H C ; Beguin S.** The effect of DDAVP infusion on thrombin generation in platelet-rich plasma of von Willebrand type 1 and in mild haemophilia A patients. *Thromb Haemost,* 2000, vol. 84, 638-42
- **Rijkers DT ; Wielders SJ ; Beguin S ; Hemker HC.** Prevention of the influence of fibrin and alpha2-macroglobulin in the continuous measurement of the thrombin potential: implications for an endpoint determination of the optical density. *Thromb. Res.,* 15 February 1998, vol. 89 (4), 161-9
- **Kessels H ; Willems G ; Hemker HC.** Analysis of thrombin generation in plasma. *Comput Biol Med,* July 1994, vol. 24 (4), 277-88
- **Beguin S ; Lindhout T ; Hemker HC.** The effect of trace amounts of Tissue Factor on thrombin generation in platelet rich plasma, its inhibition by heparin. *Thromb Haemost,* 28 February 1989, vol. 61 (1), 25-9
- **Kessels H ; Beguin S ; Andree H ; Hemker HC.** Measurement of thrombin generation in whole blood--the effect of heparin and aspirin. *Thromb Haemost,* July 1994, vol. 72 (1), 78-83
- **Reverter JC ; Beguin S ; Kessels H ; Kumar R ; Hemker HC ; Coller BS.** Inhibition of platelet-mediated, Tissue Factor-induced thrombin generation by the mouse/human chimeric 7E3 antibody. Potential implications for the effect of c7E3 Fab treatment on acute thrombosis and "clinical restenosis". *J Clin Invest,* 01 August 1996, vol. 98 (3), 863-74
- **Butenas S ; van't Veer C ; Mann KG.** "Normal" thrombin generation. *Blood,* 01 October 1999, vol. 94 (7), 2169-78
- **van 't Veer C ; Golden NJ ; Kalafatis M ; Simioni P ; Bertina RM ; Mann KG.** An in vitro analysis of the combination of haemophilia A and factor V (LEIDEN). *Blood,* 15 October 1997, vol. 90 (8), 3067-72
- **Fareed J ; Messmore HL ; Walenga JM ; Bermes EW Jr.** Synthetic peptide substrates in hemostatic testing. *Crit Rev Clin Lab Sci.,* 1983, vol. 19 (2), 71-134
- **Blomback M ; Blomback B ; Olsson P ; Svendsen L.** The assay of antithrombin using a synthetic chromogenic substrate for thrombin. *Thromb Res,* November 1974, vol. 5 (5), 621-32
- **Choo IH ; Didisheim P ; Doerge ML ; Johnson ML ; Bach ML ; Melchert LM ; Johnson WJ ; Taylor WF.** Evaluation of a heparin assay method using a fluorogenic synthetic peptide substrate for thrombin. *Thromb Res Suppl.,* 01 January 1982, vol. 25 (1-2), 115-23
- **Hitomi Y ; Kanda T ; Niinobe M ; Fujii S.** A sensitive colorimetric assay for thrombin, prothrombin and antithrombin III in human plasma using a new synthetic substrate. *Clin Chim Acta,* 12 March 1982, vol. 119 (3), 157-64
- **Griessbach U ; Sturzebecher J ; Markwardt F.** Assay of hirudin in plasma using a chromogenic thrombin substrate. *Thromb Res Suppl.,* 15 January 1985, vol. 37 (2), 347-50
- **Ohki M ; Tanaka S ; Uchida K ; Yamaguchi T ; Kato H.** A basic study on a highly sensitive automated method for hypercoagulable state in plasma, fluorogenic Prothrombin Time method. *Rinsho Byori,* October 1993, vol. 41 (10), 1153-8